# EUROPEAN PATENT APPLICATION

(11) **EP 1 477 115 A1**
(43) Date of publication of application: **17.11.2004**
(21) Application number: 04002327.7
(22) Date of filing: 03.02.2004
(51) Int. Cl.: A61B 5/055, A61B 19/00, G01R 33/28

(54) **Surgical operation apparatus**

(30) Priority: 12.05.2003 JP 2003132604
(71) Applicant: Hitachi, Ltd., Chiyoda-ku, Tokyo 101-8010 (JP)
(72) Inventor: Tajima, Fujio, c/o Hitachi, Ltd., Int. Prop. Group, Tokyo 100-8220 (JP)
(74) Representative: Beetz & Partner Patentanwälte

(57) **Abstract**

In a surgical operation apparatus to be used in a surgical operation within a body cavity, in particular, circulatory organs, respiratory organs, or abdominal cavity, a tip portion of a manipulator is operated, being inserted into a target region within an organ within a living body (104), while making monitor on imaging diagnostic equipment. This apparatus comprises: an image information detector unit (101, 103) for detecting image information within a body, including the target region therein; a manipulator mechanism unit (102) for operating the tip portion within the target region; and an operation inputting unit (116, 117) for inputting operation information therein. A controller unit (110), for controlling those, changes a condition of information detection while referring to the image information detected by the image information detector unit, and also controls the manipulator mechanism unit (102) in accordance with the operation information inputted into the operation inputting unit (116, 117), wherein a portion of the manipulator mechanism unit (102) and a portion of the image information detector unit (101, 103) are made use in common with each other.

## Description

### BACKGROUND OF THE INVENTION

The present invention relates to a surgical operation apparatus, and in particular, it relates to the surgical operation apparatus for use of assisting a medical treatment onto the seat of a disease by an operator, in a case of the surgical operation of; such as, a circulatory disease, a respiratory disease, or an abdominal disease, etc., for example.

In recent years, demands or needs come up upon measuring equipments for use in medical treatments, being high in accuracy and low in stress. In particular, with a wide use of a magnetic resonance image (hereinafter, being called by "MRI"), it is able to provide much information in the diagnosis. And, an MRI of an open-type is put into practical use thereof, with which an access can be made from an outside into an inside of a gantry, and therefore, a report begins to be made on a clinical example, with using a so-called interventional MRI (i.e., taking an image by means of the MRI, while making a medical treatment) within an inside and an outside of this country (or, domestically or world widely).

Thus, it can be said that, development is urgent necessity of a system for making use of such the MRI more effective and also for assisting the diagnosis/medical treatment, being safe much more and low in the stress, as well as, an elemental technology thereof; i.e., a manipulator for use in a surgical operation is of urgent necessity, being adapted into an MRI environment.

On the contrary to this, developments are made, aggressively, in particular on a system for operating the various equipments mentioned above, upon the basis of a robot control technology, everywhere in this country or over the world. Also, developments are made every year on a forming/machining technology of the material, which gives no interference with an image pick-up system if being disposed within the magnetic field of the MRI, such as, a ceramic or a plastic of high strength, etc., for example, and now, it is possible to adopt such the materials to be the structural material or into elemental parts thereof. Accompanying with this, an actuator is also developed, which can be used in the magnetic field, and it is in the situation that study can be made technically on a mechanism, which is operable within the magnetic filed of the MRI; i.e., it is in a situation that the MRI can be taken into the consideration, as a modality for measurement during the surgical operation.

Under such the situation, however conventionally, as a surgical operation apparatus being adapted to the MRI environment mentioned above, there is already known a manipulator for use of a surgical operation, shown in Japanese Patent Laying-Open No. 2001-198875 (2001), for example, which makes an operation by extending an arm from an outside of the MRI. This manipulator for use of the surgical operation has a link mechanism for controlling the position/posture of a tip through driving of multi-degrees of freedom at the roots or bases of two (2) pieces of longitudinal arms, which are coupled by a spherical joint at the tip, thereby enabling an achievement of five (5) degrees of freedom, with only driving at the bases thereof.

However, the link mechanism described in the Japanese Patent Laying-Open No. 2001-198875 (2001) has such a structure that, while the driving portion is disposed in an outside of the MRI, the long arms extend therefrom, approaching up to a target part within an image pickup space, and it occupies a very large place or space when positioning it in an actual place where the surgical operation is conducted, therefore it has a drawback that it lowers the work efficiency of the operator(s) and/or an operation staff(s).

To be used in the actual place where the surgical operation is conducted, for such kind of the mechanism, it is desired to be as small in sizes as possible. Furthermore, for achieving a manipulator, being very small in an interference with the magnetic field and/or the electromagnetic waves of the MRI, and being operable with safe, it is desired that the material is low in both the susceptibility and the conductivity thereof, for building up a portion, which enters into the MRI to reach the image pickup region; i.e., in general, being non-magnetic and made of an insulator, etc., (such as, a so-called engineering plastic, for example). However, the engineering plastic is lower in the strength, comparing to that of a metal material of the same shape/volume, then if the arm is designed in the same manner to the case of assuming that it is formed with using the metal material, it comes to be short in the strength/rigidity, such as, it is bent in the middle thereof if the arm is long one, or it vibrates with a large amplitude during the surgical operation thereof, for example; therefore there is a possibility that the position/posture at the tip thereof cannot be controlled, correctly.

Then, if trying to design it not to be bent, however the arm must be thick and large, then it fails to fulfill or satisfy the demand of reducing the occupation volume as small as possible within the working space. In the Japanese Patent Laying-Open No. 2001-198875 (2001), the material of the arm is selected to be a metal being low in the magnetism, in equilibrium with the structure strength thereof, therefore it can deal with the environment of the MRI to a certain degree, however no mentioning is made about a measure for dissolving such the small-sizing and the reduction of occupation volume thereof.

Also, if a force is applied onto a base portion of the manipulator, such as, due to a bump thereon by a staff or other equipment/apparatus or the like in the place where the surgical operation is conducted, or upon generation of an accidental disaster, such as, the earthquake, etc., it can be considered that the manipulator moves in a very large extent, in particular, when the structure has the long arm, therefore there is a necessity of giving a full consideration on the safety thereof.

Furthermore, in general, a surgical operation coordinate system of the manipulator and a coordinate system of the MRI image are not unified with each other; therefore, unification is achieved with preparation of a three (3) dimensional position measurement apparatus being provided separately, so as to measuring the position therewith, as well as, with using a so-called MRI marker, an image of which can be picked up by the MRI. However, it is very troublesome and has still a problem from a viewpoint of accuracy. In addition thereto, because of necessity of provision of an additional apparatus and the control thereof, it results in complexness of the system.

### SUMMARY OF THE INVENTION

An object, according to the present invention, is to provide a surgical operation apparatus, for achieving both an improvement on workability in relation to the medical treatment, in particular, within an inside of the imaging diagnosis equipment, as well as, in the periphery thereof, and reduction of the occupation volume in the place where the operation is conducted.

For accomplishing the object mentioned above, according to the present invention, there is provided a surgical operation apparatus, to be use in a surgical operation within a body cavity, in particular, circulatory organs, respiratory organs, or abdominal cavity, for operating a tip portion of a manipulator inserted into a target region within an organ within a living body while making monitor on imaging diagnostic equipment, comprising: an image information detector unit for detecting image information within a body, including said target region therein; a manipulator mechanism unit for operating the tip portion within said target region; an operation inputting unit for inputting operation information therein; and a controller unit for changing a condition of information detection while referring to the image information detected by said image information detector unit, and also for controlling said manipulator mechanism unit in accordance with the operation information inputted in said operation inputting unit, wherein a portion of said manipulator mechanism unit and a portion of said image information detector unit are made such that they can be used in common with each other.

And also, for accomplishing the object mentioned above, according to the present invention, there is further provided a surgical operation apparatus, to be use in a surgical operation within a body cavity, in particular, circulatory organs, respiratory organs, or abdominal cavity, for operating a tip portion of a manipulator inserted into a target region within an organ within a living body while making monitor on a magnetic resonance diagnostic apparatus, comprising: a bed entering into an image pick-up region of said magnetic resonance diagnostic apparatus, on which a patient lies; an image information detector unit for detecting image information within a body, including said target region therein; a manipulator mechanism unit for operating the tip portion within said target region; an operation inputting unit for inputting operation information therein; and a controller unit for changing a condition of information detection while referring to the image information detected by said image information detector unit, and also for controlling said manipulator mechanism unit in accordance with the operation information inputted in said operation inputting unit, wherein said manipulator mechanism unit attached to said bed enters into the image pick-up region of said magnetic resonance diagnostic apparatus, and is made of a material being an insulator of non-magnetism.

### BRIEF DESCRIPTION OF THE SEVERAL VIEWS OF THE DRAWING

Fig. 1 is a block diagram of a surgical operation apparatus, for showing an example according to the present invention; and
Figs. 2 to 9 are views for explaining details on each of members to be used in the surgical operation apparatus shown in Fig. 1, wherein:

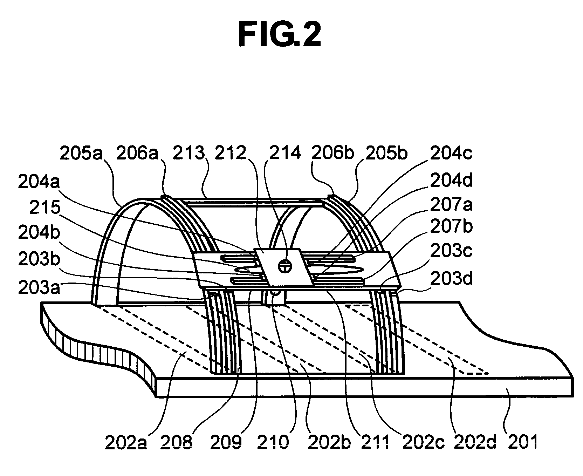

   Fig. 2 shows a perspective view of an RF coil;

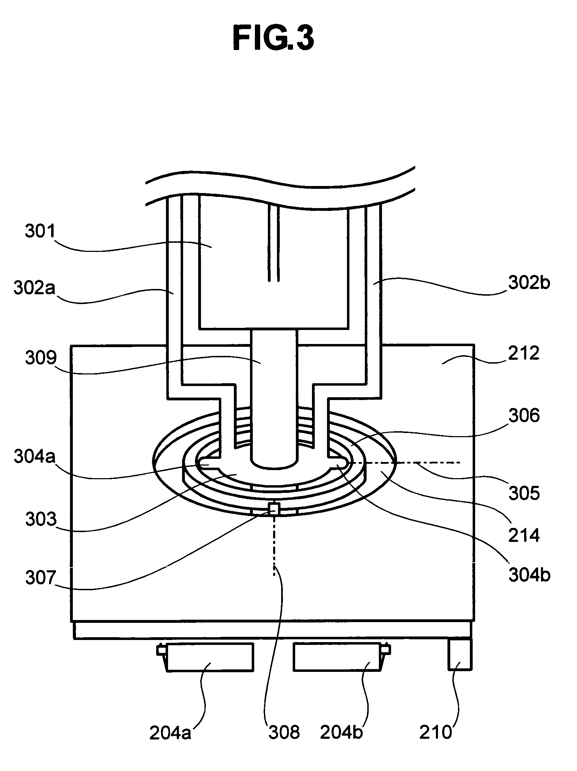

   Fig. 3 shows a perspective view of a moving gimbal of a manipulator;

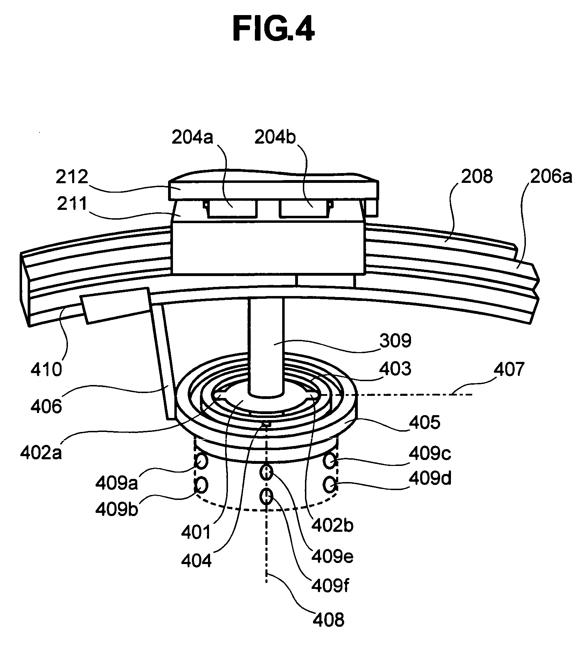

   Fig. 4 is a perspective view of a stationary gimbal thereof;

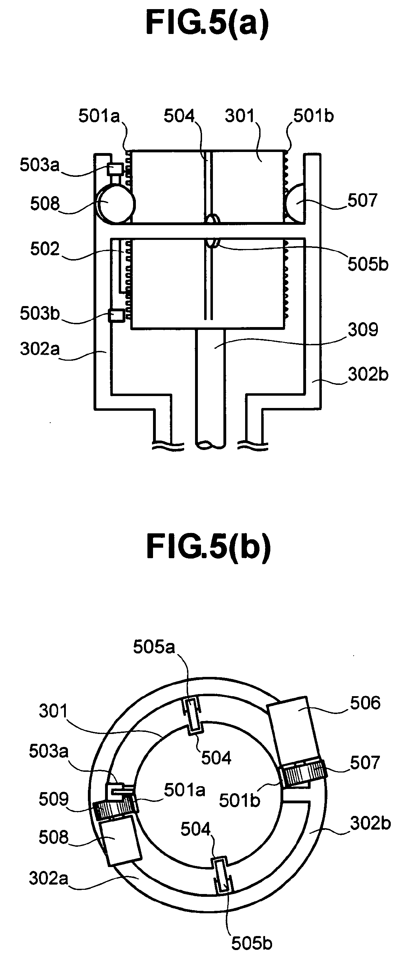

   Figs. 5 (a) and 5 (b) are up/down movement organization charts of an exchange module, and in particular, Fig. 5 (a) shows a front view, and Fig. 5(b) a plan view thereof;

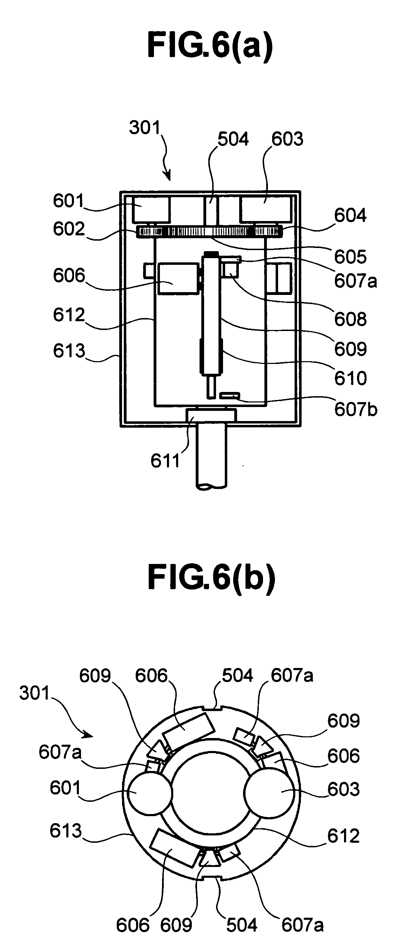

   Figs. 6 (a) and 6 (b) are views of the exchange module shown in Figs. 5 (a) and 5 (b) mentioned above, and in particular, Fig. 6 (a) shows a vertical cross-section view, and Fig. 6 (b) a horizontal cross-section view thereof;

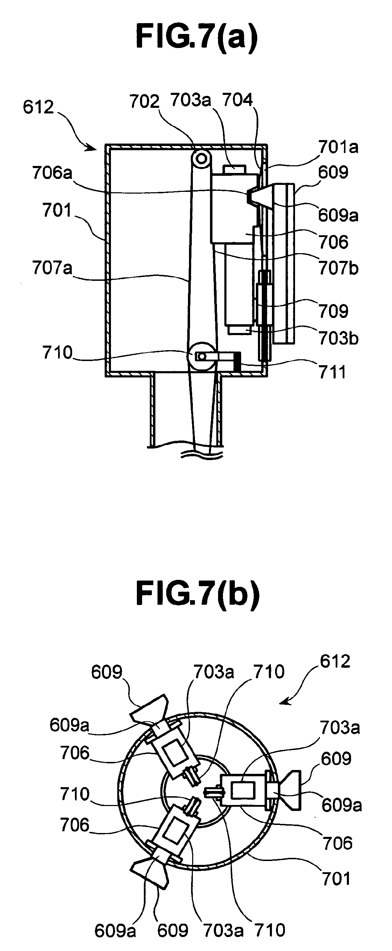

   Figs. 7(a) and 7(b) are views of a driving module of the degrees of freedom for a surgical operation tool shown in Figs. 6(a) and 6(b), and in particular, Fig. 7(a) shows a vertical cross-section view, and Fig. 7 (b) a horizontal cross-section view thereof;

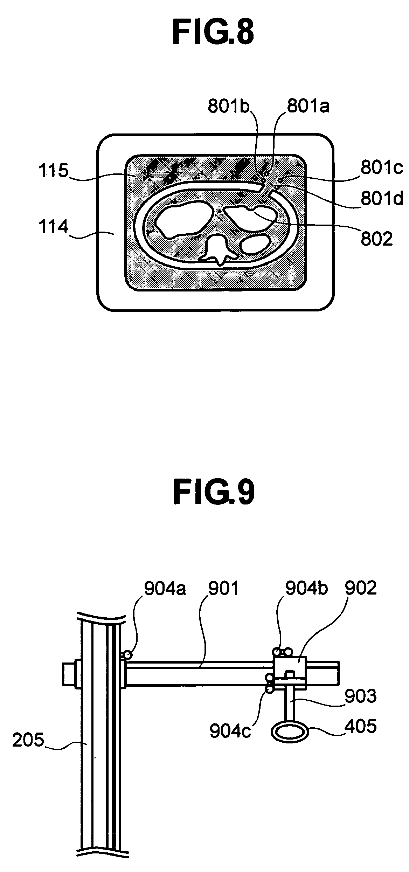

   Fig. 8 is a view for showing an example of an MRI image of an image information presentation portion; and
   Fig. 9 is a front view of an outer cylinder fixed arm for use of holding wound opened.

### DETAILED DESCRIPTION OF THE INVENTION

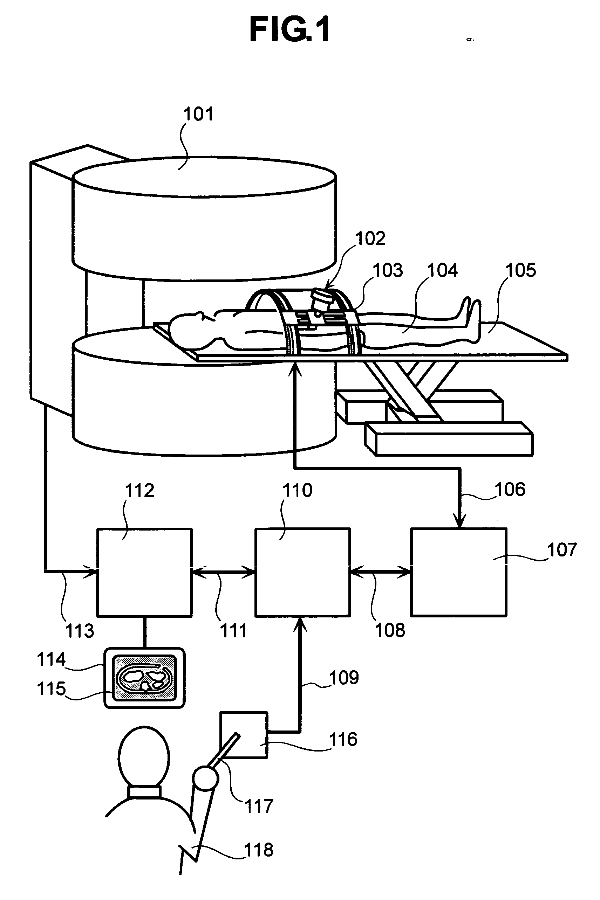

Hereinafter, explanation will be made on an embodiment of a surgical operation apparatus, according to the present invention, by referring to the drawings attached herewith. First, explanation will be given on an entire structure of the surgical operation apparatus of the present embodiment, by referring to Fig. 1. This Fig. 1 is the structure view of the surgical operation apparatus, for showing an embodiment according to the present invention.

In Fig. 1, a reference numeral 101 indicates an MRI constructing the so-called image diagnosis equipment, 102 a manipulator for use of medical treatment, 103 a coil for use of receiving resonance signal (an RF coil), 104 a patient, 105 a surgical operating table or bed, 106 a flow of actuator drive energy for each joint of the manipulator 102 and a sensor signal, as well as, the transmission path thereof, 107 a manipulator driver portion, 108 a flow of a control command and sensor information, as well as, the transmission path thereof, 109 a flow of operation information from an operation input portion 116, as well as, the transmission path thereof, 110 a controller portion, 111 a flow of diagnosis image information and image pick-up command/image pick-up condition information, as well as, the transmission path thereof, 112 an MRI controller portion, 113 a flow of a resonance signal to be an origine of image information, as well as, the transmission path thereof, 114 an image information presentation portion, 115 an image that is presented thereon, 116 an operation input portion, 117 a device for use of assistance in an operation input, and 118 is an operator.

Under the condition that the seat of a disease or the diseased part reaches to a region where an image can be picked up, while the patient 104 laying on the operation bed 105 is moved, the MRI 101 picks up an image of the diseased part of the patient 104 at an appropriate timing. The resonance signals collected therewith are processed within the MRI controller portion 112, and are presented in a form of the image 115 on the image information presentation portion 114. The operator 118, while seeing the image 115 presented thereon, makes a decision one after another, and also inputs the operation information for necessary surgical operation into the operation input portion 116, with an aid of the operation input assistance device 117. The operation input portion 116 converts the information inputted appropriately, and transmits it to the controller portion 110 as an operation command for the manipulator 102 through the transmission path 109.

The controller portion 110 manages the control of the operation apparatus, as a whole thereof. This controller portion 110 carries out a control calculation, upon the basis of the operation command from the operation input portion 116 mentioned above, the condition of the manipulator 102 at the present, and the image information from the MRI 110, and it converts a result of this control calculation into a control amount for each joint of the manipulator 102, thereby transmitting it to the manipulator driver portion 107. The manipulator driver portion 107 transmits driving energy to each join of the manipulator 102, thereby driving the each joint. The sensor information about displacement of the each joint or the like, at that instance, is detected by means of a sensor, which will be mentioned later, and is transmitted to the manipulator driver portion 107 through the transmission path 106. The manipulator driver portion 107 transmits this sensor information, further to the controller portion 110.

In those instances, a measure is taken for the manipulator driver portion 107 and the controller 110, so as not to prevent the MRI 101 from detecting the signals; i.e., an appropriate electrical isolation is made up for the manipulator driver portion 107 and the controller portion 110. For example, the manipulator driver potion 107 and the controller portion 110 are positioned in an outside of a shield room of the MRI 110, so that control/signal lines, which are shielded certainly, are reached to the manipulator 102 through a wave guide, or alternatively, an inside and an outside of the shield room are intercepted by means of a conductor panel that is grounded, thereby being so structured that the signals or the like can be transmitted through attachment of a connector hereto.

Also, it is assumed that the operation bed 105 is made of an appropriate material. For example, titanium or duralumin, or stainless steel having low susceptibility is used, properly, in a portion necessitating the strength thereon. And, it is also assumed that an engineering plastic or the like, being low in the magnetism and an insulator, is used to the utmost, in other portions thereof.

Herein, a portion of the mechanism portion of the manipulator 102 is made common in use with a portion of the RF coil 103, being an image information detection portion of the MRI 101. This commonness means not only in the structure, but also in the function thereof.

This commonness will be explained in more details thereof, by referring to Fig. 2. This Fig. 2 is a perspective view for showing an example of the structure of the RF coil 103. A portion of the RF coil 103 is made common in use with a portion of the manipulator 102. The remaining portions of those building up the manipulator 102 are not shown in this Fig. 2, forthepurposeof easy understanding thereof. However, explanation will be given on those remaining portions, later.

In Fig. 2, a reference numeral 201 depicts a top plate of a bed, 202a-202d conductors for use of receiving the resonance signals, 203a-203d an actuator for use of up/down or vertical movement along with curved rails, 204a-204d an actuator for use of horizontal movement, 205a and 205b curved portions of the coils, each enveloping the conductor for use of receiving the resonance signal therein, 206a and 206b guide rails to be used when moving vertically along with the curved portions, 207a and 207b guide rails to be used when moving horizontally, 208 a linear scale for use of detecting the vertical movement, 209 a linear scale for use of detecting the horizontal movement, 211, a vertical or up/down movement table, 212 a horizontal movement table, 213 a coupling portion of the coil enveloping the conductor for use of receiving the resonance signal therein, 214 a hole for attaching a gimbal mechanism of the manipulator, and 215 an ellipsoidal or elliptic hole. However, in some cases hereinafter, only the reference numeral is used, but omitting the alphabetic character attached therewith, in particular, when a constituent element is called by a general name, on which the reference numeral is given together with the alphabetic character.

The top plate 201 is a top portion of the operation bed 105, and is able to move reciprocally, in the horizontal direction, for example, thereby to entering into an inside of the MRI 101. Each of the portions attached with wave lines at both ends of the top plate 201 indicates that an illustration is omitted therefrom on that tip portion. Within the top plate 201 are provided the conductors 202a-202d for use of receiving the resonance signals in plural numbers thereof, as a part of the RF coil 103. Those conductors 202a-202d may be disposed horizontally, e.g. at a distance of about half (1/2) of a vertical width of coil (i.e., the distance between the curved portions of the coils). Positioning the curved portions 205a and 205b to be fitted with the positions of the conductors 202a-202d brings the conductors of the curved portions 205a and 205b to be in contact with the conductors 202a-202d, within the top plate 201. With this, the conductor 202 and the conductor 205 build up an electrical closed loop, thereby enabling to be used as a detector (i.e., a receiving antenna) of the resonance signals.

The curved portion 205 may be formed in a semi-circular shape. The up/down movement table 211 is attached, for example, onto the two (2) curved portions (205) bridging over them, while being able to move vertically along with the curved portions 205. On the curved portions 205 may be provided the guide rails for that purpose and the linear scales for use of detecting the position thereof, extending along with the curved surface. On each of the guide rails 206, there may be provided a block as a movable portion, however it is not shown in the figure herein, since it is attached on the reverse side of the up/down movement table 211.

Though not being shown in figure, either, but on contact portions with an actuator, which may be a ceramic actuator to be mentioned later, there may be pasted plates of ceramics, which are aligned with each other on the surface thereof. As the linear scale 208, such can be assumed that very fine scale marks are cut on the plate, for example, which can be read out under the condition of reflection or transmission of a light. This scale can be read out by means of a readout sensor, which may be attached on the up/down movement table 211 (this is not shown in the figure, either, since it is also on the reverse side, in the disposition thereof). Namely, the linear scale 208 and the read-out sensor thereof build up an encoder. However, as the memory scale cut thereon, it is possible to consider a means other than that mentioned above, such as, a gray code, for example.

Moreover, though not shown in the figure, however, a limit sensor may also be attached with, for the purpose of detecting upper and lower limits of a movement range of the vertical movement table 211. Driving power for vertical movement is given by means of the actuator 203 attached to the up-down movement table 211. As this can be used an actuator, such as one, which utilizes the electro-strictive effect of a ceramic, for example.

The ceramic actuator assumed herein may be in contact with a driven body on a surface thereof, and it makes a movement so that it depicts a semi-circular track by the contact surface thereof, for example. If the actuator is so provided that a tangential line of the semi-circle be in parallel with that of the guide rail in the direction thereof, then the up-down movement table moves up and down along with the curved guide rail corresponding to the movement of the actuator. This actuator may be such to be used in contact with the driven body, while applying pressurization thereon. For this reason, it has a holding torque when it stops, and therefore there is no necessity of providing a brake mechanism, separately.

On the up-down movement table 211 may be attached the guide rails 207 for moving the horizontal movement table 212 in the horizontal direction and the linear scale 209 for detecting the movement thereof. Also, for the purpose of passing through the tip portion of the manipulator, which will be mentioned later, a hole 215 which may be elliptic is opened between the two (2) pieces of guide rails 207, for example. The horizontal movement table 212 moves on the guide rails 207 in the horizontal direction. As a driving force of the horizontal movement table 212, an actuator is used herein, which may be the same to that used for driving the up/down movement table 211. In the up/down movement table 211, there is opened the hole 214 for attaching the gimbal mechanism thereto. The mechanism attached thereto will be mentioned later.

With the material of each of the constituent elements, it is accomplished as will be mentioned below, thereby keeping adaptability with the MRI. With each of the guide rails 207, the block and the rail thereof are made of using beryllium copper, a screw of titanium or brass, or the like, a ball of ceramic, and an end plate of resin, for example. Titanium and brass are low susceptibility materials, being same to beryllium copper, and the other materials are of non-magnetism and made of an insulator.

The linear scales 208 and 209 may be manufactured by making the scale marks on a glass plate, through a method, such as the evaporation or the like, for example. For reading out, an incidence light is guided from a position where no influence is given from the magnetic field and/or high frequency of the MRI, for example, through an optical fiber, and a light transmitting through it or reflected thereupon is turned back to the said position, again, through a read-out optical fiber, and this is converted into an encoder signal through an photoelectric converter circuit using a photo-transistor or the like therein. Herein, the structure is shown in the figure, in particular, of the linear encoder, but it is also possible to use a rotary-type optical fiber encoder, for the purpose of detecting the rotation of a motor or a ball screw. Or, it can be also considered to conduct the measurement of position by using a position measurement apparatus, which utilizes such a medium that will not interfere with the magnetic field and/or high frequency generated by the MRI; e.g., a light, an infrared light, a sonic wave, or a micro wave (i.e., the electromagnetic wave of frequency upper than the frequency band of the resonance signal of the MRI), for example.

To the limit sensor is guided the light also through an optical fiber, and the reflection light there upon is turned back thereto though the fiber, and thereafter, the photoelectric conversion is made thereupon in a place where no ill influence can reach thereto of the electromagnetic from the MRI. Regarding the sensor portion, such may be available that is made up with only plastics, therefore it is used herein. However, in the method for detection, it may be possible to use a light/infrared light, sonic wave, microwave, etc., in the similar manner to the linear scale mentioned above.

A ceramic actuator may be used for each of those actuators 203a-203d and 204a-204d, in the example mentioned above. The constituent elements of the each actuator include, a ceramic and a housing for receiving it therein, and a spring for applying pressurizing. The parts other than the ceramic can be made of, such as, aluminum alloy, brass, etc., for example. Herein, only that is shown, which is driven linearly, as the example, however it is also possible to use an actuator of such a kind, that it generates progressive waves on the surface of a circular-like ceramic, thereby taking out a force for rotation; e.g., so-called an ultrasonic wave motor, for example. In this case, it is also possible to make up the element parts other than the ceramics, suchas, from a metal being low in the magnetism thereof. Furthermore, it is also possible to apply an actuator therein, which does not use a driving principle due to the electromagnetic force, and there ° can be considered one of using air pressure and/or liquid pressure therein, for example.

In the conductor portion of the RF coil, copper may be used as the conductor for use of receiving the resonance signal, and others than that, materials are used therein, being non-magnetic and also of an insulator, such as, plastics, for example. However, to be used as the coil, it must be designed by taking the metal portion, such as, the guide rails mentioned above, into consideration, and in an instance, it must be adjusted in the structure, to be used as a part of the antenna portion mentioned above.

Those, such as, theup/down movement table 211, the horizontal movement table 212, and remaining mechanical portions of the manipulator, which will be mentioned later, are disposed within the magnetic field of the MRI 101 and move therein, therefore the materials of the constituent members/parts of those are selected from the non-magnetic insulators, as far as possible, so that they cause no interference upon the image pick-up operation of the MRI apparatus, nor are disturbed in the operation of the mechanical portions thereof due to the ill influences of an eddy current. As the machinery material is mainly used such one, selected from polyether ether ketones or FRP, or various kinds of machinable ceramics/engineering ceramics (however, excluding those of an alumina group therefrom, and also that including fibers of conductive materials therein). For example, as a bearing is used a ceramic bearing, and as a screw and a bolt are those made of polyether ether ketones. As the parts, which necessitate strength and are located relatively far from the magnetic field, but not so much large in the number thereof, it is possible to use aluminum alloy or brass, or titanium alloy, etc., in a part thereof.

Hereinafter, explanation will be given on each of the elements building up the remaining mechanism of the manipulator 102, one by one. Fig. 3 shows an example of the structure of a portion where the remaining mechanism is attached onto the horizontal movement table 212. In the f igure, a reference numeral 301 depicts an exchange module, 302a and 302b frames, 303 an inner ring, 304a and 304b an inner ring rotary shaft, 305 a one-dotted chain line for indicating a rotation center of the inner ring, 306 an outer ring, 307 an outer ring rotary shaft, 308 a one-dotted chain line for indicating a rotation center of the outer ring, and 309 an outer cylinder in which the surgical operation tool is inserted (hereinafter, a surgical operation tool insertion cylinder). However, the doubled-wave lines, which are shown in an upper portion in the figure, indicate that the portions above of those are omitted from illustration thereof.

The rotary shafts 304 are provided on both of the inner ring 303. The inner ring and the inner ring rotary shaft 304 rotate around the shaft center 305 with respect to the outer ring 306. And, the inner ring 303 is fixed onto the frame 302. Further, the rotary shaft 307 is fixed with respect to the outer ring 306, and it rotates around the shaft center 308 with respect to the horizontal movement table 212. The rotary shaft 307 is shown in the figure, but only one of them, however, actually, there is also provided the other shaft portion of a pair, in the portion hidden therein. Namely, a series of those elements builds up the gimbal structure of two (2) degrees of freedom. However, each of those rotary shafts 304 and 307 has a restriction force by means of a torsion spring, etc.; therefore the both inner and outer rings 303 and 306 are turned back to the horizontal direction when the surgical operation tool insertion cylinder 309 is taken or pulled out therefrom. Since this gimbal is moved in the position thereof due to the fact that each of the tables 211 and 212 is driven, therefore, this is called by a moving gimbal, hereinafter, for the convenience of the explanation thereof. Also, the inner ring 303 and the surgical operation tool insertion cylinder 309 show preferable sliding characteristics between them, therefore they will not prevent the exchange module 301 from moving up/down with respect to the frame 302, as will be mentioned later. The material of each of the parts mentioned above is selected from among the engineering plastics. In particular, on the portion of sliding is used a material, which is high in the sliding grade among the polyether ether ketones.

Next, explanation will be given on an example, in which the other one point, e.g., a fixed point in a pivoting movement, is constructed with the gimbal structure. This is located below the gimbal structure shown in Fig. 3, and is fixed on a surface of a coelom or body cavity by means of the arm extending from the RF coil. The construction thereof is same to that of the gimbal shown in Fig. 3. In the figure, a reference numeral 401 depicts an inner ring, 402a and 402b inner ring rotary shafts, 403 an outer ring, 404 an outer ring rotary shaft, 405 an outer cylinder for use of holding wound opened, 406 an arm for fixing the position and posture thereof, 407 a one-dotted chain line for indicating a rotation center of the inner ring, 408 a one-dotted chain line for indicating a rotation center of the outer ring, 409a-409f MRI markers, and 410 rail grooves for use of the fixing the outer cylinder 405.

The rotary shaft 402 is fixed to the inner ring 401. The inner ring 401 and the inner ring rotary shaft 402 can rotate around the shaft center 407 with respect to the outer ring 403. Further, the rotary shaft 404 is fixed to, with respect to the outer ring 403, but it can rotates around the shaft center 408 with respect to the outer cylinder 405 for use of holding the wound opened. Namely, a series of those elements builds up the gimbal structure of two (2) degrees of freedom. However, each of those rotary shafts 402 and 403 has a restriction force by means of a torsion spring, etc., therefore the both inner and outer rings 401 and 403 are turned back to the horizontal direction when the surgical operation tool insertion cylinder 309 is taken or pulled out. Since this gimbal is fixed to the RF coil 103, therefore, this is called by a fixed gimbal, hereinafter, for the convenience of the explanation thereof. Also, the inner ring 401 and the surgical operation tool insertion cylinder 309 show preferable sliding characteristics between them, therefore they will not prevent the exchange module 301 frommoving up/down with respect to the frame 302, in the similar manner to the inner ring 303. The material of each of the parts mentioned above is selected from among the engineering plastics. In particular, on the portion of sliding is used a material, which is high in the sliding grade among the polyether ether ketones.

Also, the outer cylinder 405 for use of holding the wound opened is inserted into the wound portion, which is opened on the surface of the body cavity. This is fixed at the position and the posture thereof by the means of the fixing arm 406, with respect to the RF coil 103. However, the degrees of freedom of the structure are three (3) in the fixing arm 406, as shown in Fig. 9; i.e., being constructed with an arm 901 moving up/down in the rail groove 410 along with the RF coil curved portion 205, a horizontal movement arm 902 extending at a right angle therefrom, and an arm 903 moving up/down into the central direction of the curved portion. Among of those, the first two (2) degrees of freedom are same to the moving direction of the up/down movement table 211 and the horizontal movement table 212 . Movement is made on the fixed gimbal, manually, in relating to those degrees of freedom. Thus, at the time when the position is determined, the fixing screws 904a-904c of the arms 901-903 are tightened up. In a portion of this outer cylinder 405 is attached with the MRI marker 409, and an image of this is picked up by the means of the MRI 101; therefore, it is possible to know at which position and/or posture the fixed gimbal lies, seeing it from the coordinate system in the image space of the MRI 101, wherein the center of magnetic field is put at the original point.

Also, as a precondition when using the MRI 101, in general, it is already known of the geometrical relationships among the center of the magnetic field and the image and the coil; i.e., the center of magnetic field of the MRI 101 is equal to the center of the coil, and it is also possible to make a correspondence, such as, a certain pixel corresponds to where in the space within an inside of the coil, on a tomogram which is picked up in a certain position/direction. For example, upon the basis of the picture or image, in which images of the markers 409a-409f are taken into, as was mentioned above, positioning can be made in advance, so that the moving gimbal is in parallel with the fixed gimbal, and also they are same in the direction of the ring hole thereof, i.e., before insertion of the exchange module.

The material of the markers 409a-409f is not specified in particular herein, however, such can be used, for example, that enclosing a food in a capsule; such as, a certain kind oils and fats, or sunflower oil, lactic oil, etc., for example. With using this, if images of four (4) pieces of the markers are taken in a picture at the maximum s ize thereof , suchas, the markers 801a-801d shown in Fig. 8, for example, then such the picture means that the image is taken by along a cross-section, cutting the outer cylinder 405 in the vertical direction. Saying it in the opposite manner, if searching out the position and the posture, thereby picking up the images including the markers 801a-801d, appropriately, at positional relationships thereof, it is possible to know the position/posture of the center of the magnetic field, or in other words, the position and the posture of the outer cylinder 405 in the coordinates fixed on the coil. Namely, the spatial position can be determined of the fixed point in the pivoting movement, and therefore with this, the position/postures of an insertion portion 802, where black color is pasted on the picture, can be calculated out from the position information of each of the joints.

With such the structure as was mentioned in the above, the exchange module 301 changes the position of the moving gimbal thereof, due to the movements of the up/down movement table 211 and the horizontal movement table 212. Accompanying with this, the exchange module declines into two (2) directions, thereby being able to make a movement swinging around the center thereof, to be the fixed point thereof, in relation to the fixed gimbal shown in Fig. 4; i.e., the so-called pivoting movement.

Next, explanation will be made on an example of the structure of the mechanism, for moving the exchange module 301 and the surgical operation tool insertion outer cylinder 309 with respect to the inner ring 401; e.g., up and down in the central shaft direction thereof, by referring to Fig. 5. In the figure, reference numerals 501a and 501b depict linear gears (i.e., racks), 502 a dog for use in detection of a movement region thereof, 503a and 503b optical sensors for detecting upper and lower limits of a movable region, 504 a guide groove for use of restricting the posture thereof, 505a and 505b guide rollers moving up and down along the guide groove 504, 506 a driver motor for use of up/down drive thereof, 507 a gear (e.g., a pinion) forming a pair together with the rack 501b, 508 an encoder, and 509 a gear (e.g., a pinion) forming a pair together with the rack 501a. However, doubled wave lines, which are shown in an upper portion in Fig. 5(a), indicate that the portions above of those are omitted from illustration thereof.

The exchange module 301 is inserted from an above portion of the frame 302, so that the gears 501a and 509, the gears 501b and 507, and the groove 504 and the guide roller 505 are engaged with, each other. In this instance, however no mistaken can be made because of the existence of the dog 502 for use of detection of the movement region. In more details thereof, first the exchange module 301 is inserted, so that the lower ends of the racks 501a and 501b on both sides of the exchange module 301 are engaged with thepinions 507 and 509, respectively. Thereafter, thedrivermotor 506 rotates at a low speed, thereby beginning to guide the exchange module 301 into the frame 302. When the exchange module 301 enters into therein at a certain degree or more than that, an upper end of the dog 502 for use of detection of the movement region passes through the optical sensor 503a. After detecting that, the motor is reversed in the rotating direction thereof at the time when the exchange module 301 further goes down a little bit therefrom, and then the driver motor 506 is stopped at the instance when the optical sensor 503a detects the dog, again, thereby setting up a count value for the encoder 508 to a reference value, such as, zero (0), for example. However, it was already explained, that the table is moved in advance, so that the moving gimbal and the fixed gimbal are aligned into the same direction in the shaft direction of the holes thereof.

When retracting (e.g., pull ing out) the exchange module 301, the operation will be made, in completely reversed manner to that mentioned in the above. Namely, first when the exchange module 301 is moved up in the shaft direction of the hole, and then if it is taken out fully, up to a front, the table is moved so that the outer and inner rings of the moving/fixed gimbals are aligned into the same direction in the shaft direction of the holes thereof, under the condition that they are in parallel to each other. Thereafter, further the driver motor 506 rotates at the low speed in such the direction that the exchange module 301 is taken out therefrom. At the time when the racks 501a and 501b and the pinions 507 and 509 come to near the end of the engagement therebetween, the lower end of the dog just passes through the optical sensor 503a; therefore, this is detected, thereby stopping the rotation of the driver motor 506.

As the method for giving an instruction upon the position/posture of the manipulator to react, there are several manners, which will be mentioned below. The easiest or simplest one of the methods is that an operator gives the instruction on the real-time base while watching the image of the MRI, from the up going mentioned above until the movement of the table. Thus, after completing the three (3) dimensional reconstructing from the image data thereon, upon the intestines and/or organs within the body, to extract a boundary surface therebetween, then this boundary surface is projected into a working space, which is corresponded thereto through the method mentioned above, so as to dissolve it as a conventional problem of avoiding an obstacle, thereby producing the position/posture instruction for the manipulator along a time series, and the control may be made in accordance with that.

In a case when inserting other exchange module 301, again, it is also possible to shorten the time up to reopening of the operations, by following the instruction data in the reverse direction, which is produced in any one of the methods when retracting. Of course, the system is so constructed, that an intervention can be made in an operation instruction depending upon the situation, while the operator always watches the operations during the time of that.

Next, explanation will be made on an example of the inner structure of the exchange module 301, by referring to Fig. 6. In the figure, a reference numeral 601 depicts an encoder for use in detection of a twisting angle, 602 a gear for use in detection of the twisting angle, 603 an actuator for use of twisting drive, 604 a gear for use in transmission of twisting drive force, 605 a gear for use in receiving the twisting drive force, 606 a ceramic linear actuator, 607a and 607b upper and lower limit sensors, 608 an optical sensor for reading out a linear scale, 609 a member of linear movement, 610 a linear guide, 611 a sliding member in twisting direction, 612 a module for driving degrees of freedom of the surgical operation tool (i.e., a surgical operation tool freedom degree drive module), and 613 an exchange module outer cylinder. The twisting angle detection encoder 601 may be made up together with other detection means; such as, a potentiometer, etc. Since a reference for selection regarding with the materials is also same to that mentioned above, therefore the exculpation thereof will be omitted herein.

The encoder 601, the actuator 603 and the sliding member 611 are fixed onto the outer cylinder 613. When the actuator 603 transmits a force through the gear 604 to the gear 605, which is connected to the surgical operation tool freedom degree drive module 612, then the module 612 rotates while twisting around a central shaft of the sliding member 611, which is made ring-like in the shape thereof. Those, from the linear actuator 606 up to the linear guide 610, are attached to the surgical operation tool freedom degree drive module 612, and therefore they change the positions, in particular, in the circumferential directions thereof, accompanying with the twisting rotation mentioned above. Those are positioned, symmetrically, by taking an every distance of 120 degrees therebetween, around the drive module 612, assuming that the degrees of freedom are three (3), as is shown in an upper cross-section view of Fig. 6(b). The linear movement member 609 is guided by means of the linear guide 610, and is driven by means of the linear actuator 606. Also, it is held at the position thereof due to the friction, in particular when the linear actuator does not operate. The upper and lower limits of the linear movement member 609 are detected by means of the limit sensors 607a and 607b. Although not shown in the figure, however change in the position thereof is detected through read-out of the liner scale attached on a side of the linear movement member 609, by means of an optical sensor.

Next, explanation will be made on an example of the inner structure of the surgical operation tool freedom degree drive module the drive module 612 (i.e., the drive module for degrees of freedom of the surgical operation tool), by referring to Fig. 7 . In the figure, a reference numeral 701 depicts an outer cylinder of the surgical operation tool freedom degree drive module, 702 is an upper portion pulley, 703a and 703b upper and lower dumpers, 704 a region separation seat, 706 a driven member, 707a and 707b wires for use of transmitting motive power to the surgical operation tool, 709 a linear guide for fixing the driven member, thereby restricting the movement thereof, but other than the upper and lower directions, 710 a tension pulley, and 711 a plate spring for applying a tension thereto.

On the outer cylinder 701, a slit 701a is opened at the position of the linear movement member 609, and into this enters the linear movement member 609, so that a convex portion 609a thereof reaches to an inside of the outer cylinder 701. The driven member 706 is positioned, so that the convex portion 609a is fitted into a concave portion 706a of itself; therefore, building up a mechanism of moving up and down accompanying with the up/down movement of the linear movement member 609. In this instance, the linear movement member 609 and the driven member 706 are separated or divided by means of the region separation seat 704, biologically. With this, since it is possible to prevent entrance and exit of microorganisms, humors, tissues, etc., therefore separation can be made between the regions, such as, a clean region and an unclean region, for example. The seat 704 is made of a material, such as, a highly polymerized compound, being soft in such a degree that it does not prevent the fitting between the linear movement member 609 and the driven member 706, but being sill hard to be torn out.

Since being restricted in the movement only in one direction by the means of the linear guide 709, the driven member 706 can make only the up and down movement; i.e., it draws up the wire 707b fixed thereto. For the purpose of removing slackness or the like in that instance, the tension pulley 710 is positioned on the way of a route of the wire 707a and 707b, being supported on the plate spring 711, so as to apply an appropriate tension thereto. Though not shown in the figure, the wire 707b is connected to the wire 707a at a tip of the surgical operation tool; i.e., the wire system is formed in a loop-like. In a case when the driven member 706 makes a movement exceeding over the moveable region due to any reason, it stops the movement since the dumper 703 bumps on the outer cylinder 701. A material, having an appropriate viscoelasticity, is used for the dumper 703, thereby to limit or absorb an impact on each other as small as possible. Further, those are also disposed at every 120 degrees, symmetrically, in the similar manner to that of the drive portion, which is attached on an outer surface of the outer cylinder 701.

In relation to the surgical operation tool, various things can be considered therein, however since they have not strong relevancy with the contents of the present invention, therefore detailed explanation thereof will be omitted. Herein, the surgical operation tool is a mechanism, having three (3) degrees of freedom at the most, and it is assumed to be the followings: such as, a forceps, a needle holder, a scissors, etc., or alternatively, an ultrasonic scalpel, a radio wave/microwave cautery probe, and a probe of guiding a low temperature material for freezing the tissues to death, etc., being lower than that in the degrees of freedom.

Regarding the materials for each of the elements, they are as was mentioned previously, and it is preferable to use the engineering plastics, such as, polyether ether ketones, mainly. Regarding the sliding portions, the material is selected from those, having a high grade in durability against sliding.

Further, fixing of the outer cylinder 405 does not have to be made onto the RF coil 103 of the MRI 101, necessarily, but it may be made onto a main body of MRI in the place thereof, for example, which can be considered to have a space coordinate system being invariant in time. However, it is necessary to know a relationship between the coordinate system of a target attached and the coordinate system of the coil, in advance, when using thereof.

Also, regarding the each of the elements for making up the manipulator 102 mentioned above, or any one of the actuators and the wires, etc., for use of driving, on which no mention was made in the above, all of them are made of the non-magnetic material, which is non-sensitive to the magnetic field. As the structure material thereof is used a non-magnetic metal, such as, duralumin (i.e., an aluminum alloy), titanium alloy or the like, or the engineering plastic. Also, in the case mentioned above, though listing up only the actuator, being built up with only the non-magnetic material but not using the electromagnetic force in the driving principle thereof; such as, the ultrasonic motor, for example, however, it is also possible to apply an actuator of liquid pressure and/or an air pressure drive, etc., in the place thereof. And, a polymeric material is used to the wire, which shows a high toughness. Portions of the surgical operation tool, which are assumed to attached to the tip thereof, they are also made of the engineering plastic or the machinable ceramics.

Summarizing the structure of the surgical operation apparatus, according to the embodiment mentioned above, are as follows:
(1) A surgical operation apparatus, to be used in a surgical operation within a body cavity, in particular, circulatory organs, respiratory organs, or abdominal cavity, for operating a tip portion of a manipulator inserted into a target region within an organ within a living body while making monitor on an imaging diagnostic equipment, comprising: an image information detector unit for detecting image information within a body, including said target region therein; a manipulator mechanism unit for operating the tip portion within said target region; an operation inputting unit for inputting operation information therein; and a controller unit for changing a condition of information detection while referring to the image information detected by said image information detector unit, and also for controlling said manipulator mechanism unit in accordance with the operation information inputted in said operation inputting unit, wherein a portion of said manipulator mechanism unit and a portion of said image information detector unit are made use in common with each other.
(2) The surgical operation apparatus, as described in the (1) mentioned above, wherein the portion of said manipulator mechanism unit and the portion of said image information detector unit are made use in common with, structurally or functionally. In the sense of the invention "structurally" may include that at least a portion of the detector for the resonance signals serves as a guiding means for a movement device of the manipulator and functionally may include that at least a portion of guiding means and/or movement means serves as a detection means.
(3) The surgical operation apparatus, as described in the (1) or (2) mentioned above, wherein the common portion of said manipulator mechanism unit is made of a material of an insulator of non-magnetism.
(4) The surgical operation apparatus, as described in the (1) mentioned above, wherein a magnetic resonance diagnostic apparatus is used as the imaging diagnostic equipment, and a signal detection portion of said image information detector unit is made use of in common with said manipulator mechanism unit.
(5) The surgical operation apparatus, as described in the (4) mentioned above, wherein a structure portion of a receiver for receiving magnetic resonance signal of said magnetic resonance diagnostic apparatus is made use of in common with said manipulator mechanism unit.
(6) The surgical operation apparatus, as described in the (5) mentioned above, wherein said receiver is constructed with a portion which is preferably curved, being formed to project upward from a bed entering into an image pick-up region of said magnetic resonance diagnostic apparatus, on which a patient is lying, and a portion provided within an inside of said bed, and both of those are shaped in loop-like.
(7) The surgical operation apparatus, as described above, wherein said receiver is constructed with a plural number of portions which are preferably curved, each being formed to project upward from a bed entering into an image pick-up region of said magnetic resonance diagnostic apparatus, on which a patient lies, and said manipulator mechanism unit comprises a movement table being provided bridging over said portions, so as to move along with said portions, a movement table being provided on said table, so as to move in a horizontal direction, and a surgical operation tool attached to said movement table through a mechanism, which is a gimbal mechanism, preferably.
(8) The surgical operation apparatus, as described in any one of the (5) to (7) mentioned above, wherein said manipulator mechanism unit is attached to said receiver through a moving gimbal of three (3) degrees of freedom, and a fixed gimbal of two (2) degrees of freedom, being positioned near to a side of the surgical operation tool than said moving gimbal.
(9) A surgical operation apparatus, to be use in a surgical operation within a body cavity, in particular, circulatory organs, respiratory organs, or abdominal cavity, for operating a tip portion of a manipulator inserted into a target region within an organ within a living body while making monitor on a magnetic resonance diagnostic apparatus, comprising: a bed entering into an image pick-up region of said magnetic resonance diagnostic apparatus, on which a patient lies; an image information detector unit for detecting image information within a body, including said target region therein; a manipulator mechanism unit for operating the tip portion within said target region; an operation inputting unit for inputting operation information therein; and a controller unit for changing a condition of information detection while referring to the image information detected by said image information detector unit, and also for controlling said manipulator mechanism unit in accordance with the operation information inputted in said operation inputting unit, wherein said manipulator mechanism unit attached to said bed enters into the image pick-up region of said magnetic resonance diagnostic apparatus, and is made of a material an insulator of non-magnetism.
(10) The surgical operation apparatus, as described in the (9) mentioned above, wherein said manipulator mechanism unit is attached to the receiver for receiving magnetic resonance signal of said magnetic resonance diagnostic apparatus.
(11) The surgical operation apparatus, as described in the (10) mentioned above, wherein said manipulator mechanism unit is attached to said receiver through a moving gimbal of three (3) degrees of freedom, and a fixed gimbal of two (2) degrees of freedom, being positioned near to a side of the surgical operation tool than said moving gimbal.

The surgical operation apparatus, being constructed in such a manner as was mentioned above, has the following advantages:
(1) Making the manipulator and at least a portion of the structure/mechanism of the imaging diagnostic equipment common in use can achieve a small/compact sizing of the manipulator mechanism.
(2) Making the signal detector unit of the diagnostic equipment and the portion of the manipulator mechanism, common in use thereof, structurally/functionally, enables unification of the coordinates with using the image information, but without necessity of including an additional apparatus, such as, a position measurement apparatus, etc., in the constituent elements thereof, thereby contributing to keep the system scale to be small.

In the sense of the invention "structurally" means such an aspect that a portion of the RF coil, which is preferably curved, is commonly used, as a guiding means, such as a rail for a movement device, for example, and "functionally" means that a portion of the guiding means makes up a portion of the RF signal receiver, etc.

Also, the mechanism structure, being based on the pivoting movement in a narrow region, brings about the following advantages:
(3) Driving the base position by the driving mechanism, which is restricted in the movable region thereof, and also restricting the movement with provision of a fulcrum on the way thereof, enable to avoid mechanically a dangerous malfunction therefrom, such as, swinging the long insertion portion.
(4) Provision of the structure for supporting a middle of the insertion portion reduces the bending in a tip thereof, thereby increasing safety and/or reliability as the manipulator for use in the surgical operation.
(5) Transmitting the driving force through a wire, etc., while positioning the driving source of the tip portion at the base thereof, enables to achieve a locally complicated operation of the manipulator, in spite of a fact that the manipulator does not move so much as a whole for driving of the tip portion thereof.
(6) Appling a linear movement mechanism into position movement of the base makes the movement of each of the manipulator intutive and/or easily understandable. With this, safety can be easily obtained for an operation working around it. Also, it is the structure of being high in affinity with stereotaxic surgical operation.
(7) As was mentioned in the above, according to the surgical operation apparatus of the present invention, the movement of the manipulator, as a whole thereof, is not so large, and it also has the structure and degrees of freedom, enabling complicated operation at the tip portion thereof, locally, therefore being suitable for the operation of such the surgical operation within a narrow space of the MRI.

And, it is also one of the advantages that, not only the tip portion thereof, but also the entire movement of the mechanism can be made within a small region. Namely,
(8) It is possible to suppress the operation region and the operation speed small at the base thereof, for achieving the tip operation. With this, it is possible to obtain the safety of the operator around it.

As was fully explained in the above, according to the present invention, it is possible to provide the surgical operation apparatus, enabling to achieve both; i.e., an improvement on operability, in particular, in relation to the medical treatment conducted in an inside and in periphery of the image diagnostic equipment, and also reduction of the occupation volume thereof in a place where the surgical operation is conducted.

The present invention may be embodied in other specific forms without departing from the spirit or essential feature or characteristics thereof. The present embodiment(s) is/are therefore to be considered in all respects as illustrative and not restrictive, the scope of the invention being indicated by the appended claims rather than by the forgoing description and range of equivalency of the claims are therefore to be embraces therein.

## Claims

1. A surgical operation apparatus for operating a tip portion of a manipulator inserted into a target region within an organ within a living body while making monitor on imaging diagnostic equipment (101, 103), comprising:
an image information detector unit (101, 103) for detecting image information within a body, including said target region therein;
a manipulator mechanism unit (102) for operating the tip portion within said target region;
an operation inputting unit (117, 116) for inputting operation information therein; and
a controller unit (110) for changing a condition of information detection while referring to the image information detected by said image information detector unit (101, 103), and also for controlling said manipulator mechanism unit (102) in accordance with the operation information inputted in said operation inputting unit (116, 117), wherein a portion of said manipulator mechanism unit (102) and a portion of said image information detector unit (101, 103) are made use in common with each other.

2. Surgical operation apparatus according to claim 1, **characterized in that** the portion of said manipulator mechanism unit (102) and the portion of said image information detector unit (101, 103) are made such that they are used in common with, structurally or functionally.

3. Surgical operation apparatus according to claim 1 or 2, **characterized in that** the common portion of said manipulator mechanism unit (102) is made of a material of an insulator of non-magnetism.

4. Surgical operation apparatus according to any of the preceding claims, **characterized in that** a magnetic resonance diagnostic apparatus is used as the imaging diagnostic equipment, and a signal detection portion of said image information detector unit (101, 112) is made use in common with said manipulator mechanism unit (102, 103).

5. Surgical operation apparatus according to any of the preceding claims, **characterized in that** a structure portion of a receiver for receiving magnetic resonance signal of said magnetic resonance diagnostic apparatus is made use in common with said manipulator mechanism unit (102, 103).

6. Surgical operation apparatus according to any of the preceding claims, **characterized in that** a receiver is constructed with a portion being formed to project upward from a bed entering into an image pick-up region of said magnetic resonance diagnostic apparatus, on which a patient is lying, and a portion provided within an inside of said bed, and both of those are shaped in loop-like.

7. Surgical operation apparatus according to claim 5, **characterized in that** said receiver is constructed with a plural number of portions, each being formed to project upward from a bed entering into an image pick-up region of said magnetic resonance diagnostic apparatus, on which a patient lies, and said manipulator mechanism unit comprises first movement means being provided bridging over said portions, so as to move along with said portions, second movement means being provided on said first means, and a surgical operation tool attached to said second movement means.

8. Surgical operation apparatus according to claim 7, **characterized in that** said first movement means comprises an up/down table and said second movement means comprises a horizontal movement table.

9. Surgical operation apparatus according to any of claims 5-8, **characterized in that** said manipulator mechanism unit (102) is attached to said receiver through a moving gimbal of three (3) degrees of freedom, and a fixed gimbal of two (2) degrees of freedom, being positioned near to a side of the surgical operation tool than said moving gimbal.

10. A surgical operation apparatus for operating a tip portion of a manipulator inserted into a target region within an organ within a living body while making monitor on a magnetic resonance diagnostic apparatus, comprising:
a bed (105) entering into an image pick-up region of said magnetic resonance diagnostic apparatus, on which a patient can lie,
an image information detector unit (101, 112) for detecting image information within a body (104), including said target region therein;
a manipulator mechanism unit (102) for operating the tip portion within said target region;
an operation inputting unit (116, 117) for inputting operation information therein; and
a controller unit (110) for changing a condition of information detection while referring to the image information detected by said image information detector unit (101, 112), and also for controlling said manipulator mechanism unit (102) in accordance with the operation information inputted in said operation inputting unit (116, 117), wherein said manipulator mechanism unit (102) attached to said bed enters into the image pick-up region of said magnetic resonance diagnostic apparatus, and is made of a material an insulator of non-magnetism.

11. Surgical operation apparatus according to claim 10, **characterized in that** said manipulator mechanism unit (102) is attached to the receiver for receiving magnetic resonance signal of said magnetic resonance diagnostic apparatus.

12. Surgical operation apparatus according to claim 10 or 11, **characterized in that** said manipulator mechanism unit (102) is attached to said receiver through a moving gimbal of three (3) degrees of freedom, and a fixed gimbal of two (2) degrees of freedom, being positioned near to a side of the surgical operation tool than said moving gimbal.
